# EUROPÄISCHE PATENTSCHRIFT

(11) **EP 2 082 778 B1**
(45) Veröffentlichungstag und Bekanntmachung des Hinweises auf die Patenterteilung: **10.08.2011**
(21) Anmeldenummer: 09000420.1
(22) Anmeldetag: 14.01.2009
(51) Int. Cl.: A61N 1/36

(54) **Bipolare Elektrode**
Bipolar electrode
Electrode bipolaire

(30) Priorität: 22.01.2008 DE 102008005377
(43) Veröffentlichungstag der Anmeldung: 29.07.2009
(73) Patentinhaber: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(56) Entgegenhaltungen:
- EP-A- 1 709 990
- WO-A-00/32130
- WO-A-98/17346
- DE-B3-102005 015 487
- US-A1- 2003 023 295
- US-A1- 2008 039 896

## Beschreibung

Die Erfindung betrifft eine bipolare Elektrode mit zwei zueinander beabstandeten Polpaaren, die jeweils aus einer Kathode und einer Anode gebildet sind, wobei das eine Polpaar in Gebrauchsstellung im Ventrikel eines Herzens und das andere Polpaar in dessen Vorhof angeordnet sind.

Derartige Elektroden, zum Beispiel Herzschrittmacherelektroden, sind bekannt und müssen aufgrund der unterschiedlichen Anatomie der verschiedenen Herzpatienten in unterschiedlichen Abmessungen auf Vorrat gehalten werden.

Aus der US-A1-2003/023295 ist eine Elektrode mit zwei ineinander steckbaren Leitungen bekannt, die für ihre Funktion ineinander in eine Gebrauchsstellung verschiebbar und in dieser festlegbar, dann aber nicht mehr verstellbar sind. Somit müssen auch von einer derartigen Elektrode unterschiedliche Abmessungen für verschiedene Herzpatienten auf Vorrat gehalten werden.

Es besteht deshalb die Aufgabe, eine Elektrode der eingangs definierten Art zu schaffen, die für praktisch alle denkbaren unterschiedlichen Abmessungen oder anatomischen Gegebenheiten nutzbar ist.

Zur Lösung dieser Aufgabe ist vorgesehen, dass das in dem Ventrikel platzierbare Polpaar an einem ersten Schaft oder Schlauch und das zweite, im Vorhof einsetzbare Polpaar an einem zweiten Schaft oder Schlauch angeordnet sind und dass die beiden Schäfte oder Schläuche in axialer Richtung relativ zueinander verschiebbar oder verstellbar und dadurch die Abstände der beiden Polpaare zur Anpassung an unterschiedliche anatomische Gegebenheiten wählbar oder einstellbar sind, wobei der zweite Schaft den ersten Schaft bereichsweise außenseitig umschließt und dass die beiden jeweils wenigstens ein Polpaar tragenden Schäfte oder Schläuche konzentrisch zueinander angeordnet sind und der äußere Schaft das oder die Polpaare für den Einsatz im Vorhof trägt.

Auf diese Weise wird eine bipolare Elektrode zur Verfügung gestellt, bei welcher der Abstand der Polpaare auf die jeweiligen unterschiedlichen anatomischen Gegebenheiten verschiedener Herzpatienten eingestellt werden kann, so dass keine unterschiedlich bemessenen Elektroden auf Vorrat gehalten werden müssen. Dabei kann für die Änderung des Abstands der Polpaare der äußere Schaft relativ zu dem innenliegende Schaft in dessen Längserstreckungsrichtung verschoben werden, wobei der innenliegende erste Schaft mit seinen festliegenden Polpaar im Ventrikel platziert und dann der zweite Schaft für die entsprechende anatomische Gegebenheit so verschoben werden kann, dass das oder die von ihm getragenen Polpaare im Vorhof in zutreffender Form platziert werden.

Zwar ist aus der US 2006/0064150 A1 eine teleskopartig verschiebbare Elektrodenanordnung bekannt, bei welcher zwei Elektroden relativ zueinander verstellbar sind, jedoch trägt jede Elektrode nur einen Pol, so dass diese vorbekannte Anordnung nicht zur Anwendung im Ventrikel eines Herzens einerseits und im Vorhof dieses Herzens andererseits vorgesehen und geeignet ist.

Günstig ist es dabei, wenn die beiden Schäfte relativ zueinander festlegbar sind und zur gegenseitigen Fixierung am proximalen Ende des äußeren zweiten Schafts eine insbesondere hülsenartige Kupplung vorgesehen ist, die mit dem innenliegenden ersten Schaft, diesen zumindest teilweise umschließend, verbindbar oder verklemmbar ist. Dies stellt eine sehr einfache Konstruktion dar, mit der nicht nur die Herstellung und Montage, sondern vor allem auch die Handhabung einfach durchführbar sind.

Vorteilhaft kann es sein, wenn am proximalen Ende die beiden Schäfte flüssigkeitsdicht miteinander verbunden sind. Somit kann ein Blutfluss zwischen den beiden Schäften vermieden werden.

Die Abdichtung zur flüssigkeitsdichten Verbindung der beiden Schäfte kann im Bereich der Kupplungshülse in deren Innerem angeordnet sein, so dass die Kupplungshülse eine Zusatzfunktion dadurch erhält, dass sie diese Dichtung aufnimmt.

Zum Fixieren der relativen Lage der beiden Schäfte oder Schläuche in Gebrauchsstellung zueinander kann wenigstens eine Klemmschraube vorgesehen sein. Dies stellt ein besonders einfach zu handhabendes Element dar, mit welchem die zunächst einzustellende relative Lage der beiden Schäfte oder Schläuche und damit der jeweiligen Polpaare fixiert werden kann.

Eine weitere Ausgestaltung der Erfindung kann vorgesehen, dass der erste Schaft eine Markierung aufweist, relativ zu welcher der zweite Schaft kontrolliert in Längsrichtung verstellbar und einstellbar ist. Dadurch kann das Längenmaß der gegenseitigen Relativverstellung kontrolliert und schneller gefunden werden.

Der zweite äußere Schaft kann relativ zu dem ersten Schaft verdrehbar sein.

Da die beiden Schäfte relativ zueinander verschiebbar sind, können sie auch sehr einfach relativ zueinander verdrehbar gemacht werden oder sein, so dass die Lage der Pole der Polpaare nicht nur durch den gegenseitigen Abstand, sondern auch die Anordnung relativ zum Umfang der Elektrode gewählt und eingestellt werden kann.

Aufgrund der relativen Verschiebbarkeit der beiden Schäfte ist eine besonders vorteilhafte und zweckmäßige Ausgestaltung dahingehend möglich, das am distalen Ende des ersten Schafts eine Verankerung, insbesondere eine Schraubwendel, angeordnet ist oder vorsteht, die von dem dabei vorgeschobenen zweiten Schaft beim Implantieren umschlossen und nach dem Einführen in das Herz durch Zurückziehen des zweiten Schafts oder Schlauches freilegbar ist. Somit kann die mögliche Relativbewegung zwischen den beiden Schäften zusätzlich dazu ausgenutzt werden, einen Anker oder eine Schraubwendel am distalen Ende des ersten Schafts während der Implantation zu umhüllen und dadurch die Blutgefäße gegen eine Verletzungsgefahr zu schützen. Auch eine Verhakung von stift- oder widerhakenartigen Verankerungsteilen könnte auf diese Weise während der Implantation vermieden werden.

Vor allem bei Kombination einzelner oder mehrerer der vorbeschriebenen Merkmale und Maßnahmen ergibt sich eine bipolare Elektrode mit zueinander beabstandeten Polpaaren, deren eines im Ventrikel und deren anderes im Vorhof eines Herzens wirksam gemacht werden können sollen, wobei die Elektrode durch eine relative Verschiebbarkeit der Polpaare in Längs- oder Axialrichtung oder in Richtung der Längsachse der Elektrode an unterschiedliche anatomische Verhältnisse angepasst werden können, so dass vermieden werden kann, unterschiedlich bemessene Elektroden auf Vorrat halten zu müssen.

Nachstehend ist ein Ausführungsbeispiel der Erfindung anhand der Zeichnung näher beschrieben. Es zeigt in zum Teil schematisierter Darstellung:
- Fig. 1: eine Seitenansicht einer erfindungsgemäßen Elekt- rode mit zwei in Längsrichtung relativ zueinander verschiebbaren Schäften, die jeweils wenigstens ein aus Anode und Kathode bestehendes Polpaar tragen, wobei der außenliegende Schaft relativ zu dem in- nenliegenden Schaft noch vorgeschoben ist für die Einführphase dieser Elektrode und wobei im proxima- len Bereich an dem innenliegenden Schaft eine Mar- kierung für die Einstellung des gegenseitiges Ab- stands der Polpaare erkennbar ist,
- Fig. 2: eine der Fig. 1 entsprechende Darstellung nach dem Zurückziehen der im Vorhof zu platzierenden Pole relativ zu dem innenliegenden Schaft und relativ zu der Markierung sowie
- Fig. 3: eine der Fig. 2 entsprechende Darstellung mit einem abgewandelten äußeren Schaft, der relativ zu dem innenliegenden Schaft drehbar ist, wobei die im Vorhof zu platzierenden Pole um 180° zueinander versetzt an diesem Schaft vorgesehen sind.

Eine im Ganzen mit 1 bezeichnete bipolare Elektrode weist zwei zueinander beabstandete, jeweils aus einer Kathode und einer Anode bestehende Polpaare 2 und 3 auf, wobei das eine Polpaar 2 in Gebrauchsstellung im Ventrikel eines Herzens und das andere Polpaar 3 in dessen Vorhof angeordnet sein sollen. Die Polpaare 2 und 3 müssen also einen jeweils der Anatomie des zu behandelten Herzens entsprechenden Abstand zueinander haben.

Damit für eine derartige Behandlung nicht zahlreiche Elektroden 1 unterschiedlicher Abmessungen notwendig sind, sind das in dem Ventrikel platzierbare Polpaar 2 an einen ersten Schaft 4 oder Schlauch und das zweite, im Vorhof einsetzbare Polpaar 3 an einen zweiten Schaft 5 oder Schlauch angeordnet und die beiden Schäfte 4 und 5 sind, wie der Vergleich der Fig. 1 und 2 oder der Fig. 1 und 3 verdeutlicht, in axialer Richtung relativ zueinander verschiebbar oder verstellbar, wodurch die Abstände der beiden Polpaare 2 und 3 wählbar oder einstellbar sind. Dabei umschließt der zweite Schaft 5 den ersten Schaft 4 außenseitig und die beiden jeweils wenigstens ein Polpaar tragenden Schäfte sind konzentrisch zueinander angeordnet und in den Figuren erkennt man, dass dabei der äußere Schaft 5 das oder die Polpaare 3 für den Einsatz im Vorhof trägt.

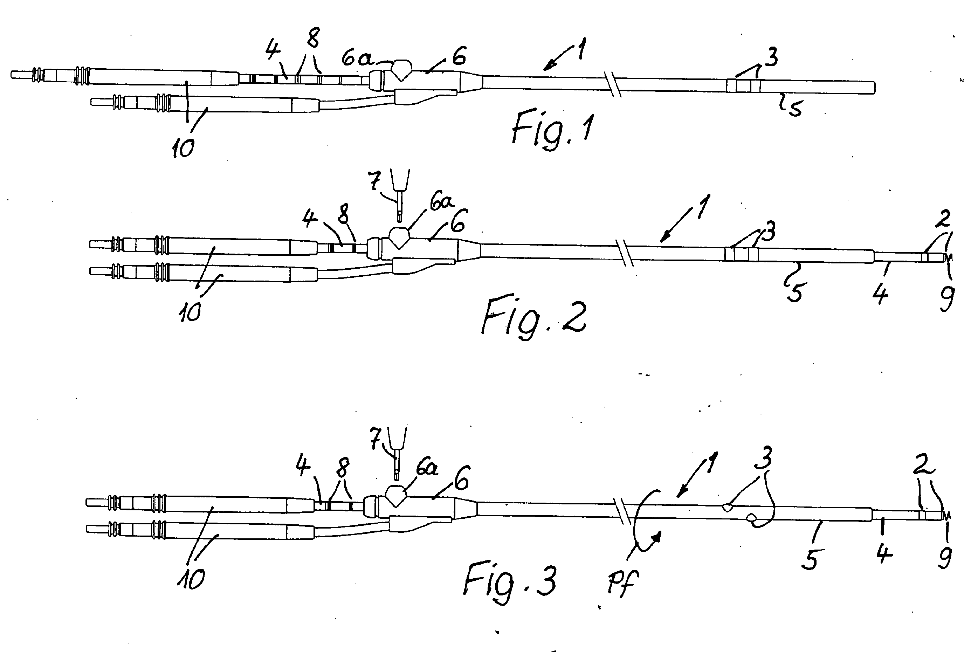

Die relativ zueinander verschiebbaren beiden Schäfte können gegenseitig festlegbar und zur gegenseitigen Fixierung am proximalen Ende des äußeren zweiten Schafts 5 eine hülsenartige Kupplung 6 haben, die mit dem innenliegenden ersten Schaft 4, diesen umschließend, verbindbar oder verklemmbar ist. Gleichzeitig kann am proximalen Ende eine flüssigkeitsdichte Verbindung der beiden Schäfte 4 und 5 vorgesehen sein, um in diesen einen Blutfluss auszuschließen. Dabei kann die Abdichtung der beiden Schäfte im Bereich der Kupplungshülse 6 in deren Inneren angeordnet sein und die relative Lage der beiden Schäfte 4 und 5 in ihrer Gebrauchsstellung kann mit wenigstens einer Klemmschraube in einem entsprechenden Stutzen 6a erfolgen, was durch das Schraubwerkzeug 7 in Fig. 2 und 3 angedeutet ist.

Vor allem in Fig. 1, aber auch in den Fig. 2 und 3 erkennt man, dass der erste Schaft 4 eine Markierung 8 aus beabstandeten Markierungsringen unterschiedlicher Anzahl aufweist, relativ zu welcher der zweite Schaft 5 und dessen distales Ende bzw. das Ende der Kupplungshülse 6 kontrolliert in Längsrichtung verstellbar und einstellbar ist. Somit kann der Abstand der Polpaare 2 und 3 anhand dieser Markierung 8 vom Benutzer bequem in einer zuvor festgelegten Weise eingestellt und fixiert werden.

Anhand der Fig. 3 ist durch den Pfeil PF angedeutet, dass der zweite äußere Schaft 5 relativ zu dem ersten Schaft 4 verdrehbar sein kann und dass die das Polpaar 3 bildeten Vorhofelektroden oder -pole um 180° zueinander versetzt sind, um bestmögliche Reizleitungen und Potentiale zu erreichen.

Beim Vergleich der Fig. 2 und 3 mit Fig. 1 wird außerdem deutlich, dass am distalen Ende des ersten Schafts 4 eine Verankerung 9 in Form einer Schraubwendel angeordnet ist oder vorsteht, die von dem dabei gemäß Fig. 1 zunächst vorgeschobenen zweiten Schaft 5 beim Implantieren umschlossen sein kann und nach dem Einführen in das Herz durch Zurückziehen des zweiten Schafts 5 freigegeben wird, um ihrerseits dann verankert zu werden. Die Verschiebbarkeit des außenliegenden zweiten Schafts 5 in Längsrichtung des ersten Schafts 4 erlaubt also nicht nur die Einstellung unterschiedlicher Abstände der von den Schäften 4 und 5 getragenen Hohlpaare 2 und 3, sondern erleichtert auch das Implantieren einer Elektrode 1 mit einer Verankerung an ihrem distalen Ende, weil diese Verankerung 9 vor allem dann, wenn sie als Schraubwendel ausgebildet ist, solange umschlossen werden kann, wie sie durch Blutgefäße und in das Herz hinein bewegt werden muss.

Am proximalen Ende der bipolaren Elektrode 1 erkennt man die beiden Stecker 10 für die beiden jeweils wenigstens ein Polpaar aufweisenden Schäfte 4 und 5 für deren Anschluss an einen Schrittmacher.

Die bipolare und implantierbare Elektrode 1 weist wenigstens zwei zueinander beabstandete Polpaare 2 und 3 auf, die jeweils aus einer Kathode und einer Anode bestehen, wobei das eine Polpaar 2 in Gebrauchsstellung im Ventrikel und das andere Polpaar im Vorhof eines Herzens angeordnet sind. Dabei ist das im Ventrikel platzierbare Polpaar 2 relativ zu dem im Vorhof einsetzbaren Polpaar 3 hinsichtlich seines Abstandes verstellbar, so dass eine Anpassung an unterschiedliche Anatomien möglich ist, also nicht eine Vielzahl von verschieden bemessenen Elektroden 1 auf Vorrat gehalten werden muss.

## Patentansprüche

1. Bipolare Elektrode (1) mit zwei zueinander beabstandeten Polpaaren, die jeweils aus einer Kathode und einer Anode gebildet sind, wobei das eine Polpaar (2) in Gebrauchsstellung im Ventrikel eines Herzens und das andere Polpaar (3) in dessen Vorhof angeordnet sind, **dadurch gekennzeichnet, dass** das in dem Ventrikel platzierbare Polpaar (2) an einem ersten Schaft (4) und das zweite, im Vorhof einsetzbare Polpaar (3) an einem zweiten Schaft (5) angeordnet sind und dass die beiden Schäfte in axialer Richtung relativ zueinander verschiebbar oder verstellbar und dadurch die Abstände der beiden Polpaare (2, 3) zur Anpassung an unterschiedliche anatomische Gegebenheiten wählbar oder einstellbar sind, wobei der zweite Schaft (5) den ersten Schaft (4) bereichsweise außenseitig umschließt, dass die beiden jeweils wenigstens ein Polpaar tragenden Schäfte konzentrisch zueinander angeordnet sind und der äußere Schaft (5) das oder die Polpaare (3) für den Einsatz im Vorhof trägt und dass am distalen Ende des ersten Schafts (4) eine Verankerung (9) in Form einer Schraubwendel angeordnet ist oder vorsteht, die von dem beim Implantieren vorgeschobenen zweiten Schaft (5) umschlossen und nach dem Einführen in das Herz durch Zurückziehen des zweiten Schafts (5) freigebbar ist.

2. Elektrode nach Anspruch 1, **dadurch gekennzeichnet, dass** die beiden Schäfte relativ zueinander festlegbar sind und zur gegenseitigen Fixierung am proximalen Ende des äußeren zweiten Schafts (5) eine insbesondere hülsenartige Kupplung (6) vorgesehen ist, die mit dem innenliegenden ersten Schaft (4), diesen zumindest teilweise umschließend, verbindbar oder verklemmbar ist.

3. Elektrode nach einem der Ansprüche 1 oder 2, **dadurch gekennzeichnet, dass** am proximalen Ende die beiden Schäfte (4, 5) flüssigkeitsdicht miteinander verbunden sind.

4. Elektrode nach einem der Ansprüche 1 bis 3, **dadurch gekennzeichnet, dass** die Abdichtung zur flüssigkeitsdichten Verbindung der beiden Schäfte im Bereich der Kupplungshülse (6) in deren Inneren angeordnet ist.

5. Elektrode nach einem der Ansprüche 1 bis 4, **dadurch gekennzeichnet, dass** zum Fixieren der relativen Lage der beiden Schäfte (4, 5) in Gebrauchsstellung zueinander wenigstens eine Klemmschraube vorgesehen ist.

6. Elektrode nach einem der Ansprüche 1 bis 5, **dadurch gekennzeichnet, dass** der erste Schaft (4) eine Markierung (8) aufweist, relativ zu welcher der zweite Schaft (5) kontrolliert in Längsrichtung verstellbar und einstellbar ist.

7. Elektrode nach einem der Ansprüche 1 bis 6, **dadurch gekennzeichnet, dass** der zweite äußere Schaft (5) relativ zu dem ersten Schaft (4) verdrehbar ist.

## Claims

1. Bipolar electrode (1) having two pairs of poles spaced from one another, each formed by a cathode and an anode, one pair of poles (2) in the position of use being disposed in the ventricle of a heart and the other pair of poles (3) being disposed in the atrium thereof, **characterised in that** the pair of poles (2) adapted to be placed in the ventricle are disposed on a first shaft (4) and the second pair of poles (3) adapted to be inserted in the atrium are disposed on a second shaft (5) and the two shafts are slidable or movable relative to one another in the axial direction and in this way the spacings of the two pairs of poles (2, 3) can be selected or adjusted to suit different anatomical conditions, the second shaft (5) partially surrounding the outside of the first shaft (4), **In that** the two shafts each carrying at least one pair of poles are arranged concentrically with respect to each other and the outer shaft (5) carries the pair or pairs of poles (3) for insertion in the atrium, and **in that** an anchorage (9) in the form of a turn of a screw is provided or protrudes at the distal end of the first shaft (4), said anchorage being surrounded by the second shaft (5) which is advanced during implantation and after being inserted in the heart it can be exposed by withdrawal of the second shaft (5).

2. Electrode according to claim 1, **characterised in that** the two shafts can be fixed relative to one another and a more particularly sleeve-like coupling (6) is provided for mutual fixing at the proximal end of the outer second shaft (5), said coupling being adapted to be connected or clamped to the inner first shaft (4), thereby at least partially surrounding the latter.

3. Electrode according to one of claims 1 or 2, **characterised in that** the two shafts (4, 5) are connected to each other in fluid-tight manner at the proximal end.

4. Electrode according to one of claims 1 to 3, **characterised in that** the seal for connecting the two shafts in fluid-tight manner is arranged in the region of the coupling sleeve (6), in the interior thereof.

5. Electrode according to one of claims 1 to 4, **characterised in that** at least one clamping screw is provided for fixing the relative position of the two shafts (4, 5) to one another in the position of use.

6. Electrode according to one of claims 1 to 5, **characterised in that** the first shaft (4) has a marking (8) relative to which the second shaft (5) is movable and adjustable in the longitudinal direction in controlled manner.

7. Electrode according to one of claims 1 to 6, **characterised in that** the second outer shaft (5) is rotatable relative to the first shaft (4).

## Revendications

1. Electrode bipolaire (1) comportant deux paires de pôles distantes l'une de l'autre, formées à chaque fois d'une cathode et d'une anode, une paire de pôles (2) étant disposée, en position d'utilisation, dans le ventricule d'un coeur et l'autre paire de pôles (3) dans son oreillette, **caractérisée en ce que** la paire de pôles (2) qui peut être placée dans le ventricule est disposée sur une première tige (4) et la deuxième paire de pôles (3) qui peut être placée dans l'oreillette sur une deuxième tige (5) et que les deux tiges sont déplaçables ou réglables l'une par rapport à l'autre en direction axiale et de ce fait les distances des deux paires de pôles (2, 3) peuvent être choisies ou réglées pour être adaptées à différentes conditions anatomiques, la deuxième tige (5) entourant en partie la première tige (4) extérieurement, que les deux tiges portant à chaque fois au moins une paire de pôles sont disposées concentriquement l'une par rapport à l'autre et la tige extérieure (5) porte la ou les paire(s) de pôles (3) destinée(s) à être placée(s) dans l'oreillette et qu'à l'extrémité distale de la première tige (4) est disposé ou fait saillie un ancrage (9) sous la forme d'une spirale de fixation qui est entourée par la deuxième tige (5) poussée en avant lors de l'implantation et qui peut être dégagée par retrait de la deuxième tige (5) après introduction dans le coeur.

2. Electrode selon la revendication 1, **caractérisée en ce que** les deux tiges peuvent être fixées l'une par rapport à l'autre et qu'il est prévu, pour la fixation mutuelle, à l'extrémité proximale de la deuxième extérieure tige (5), un accouplement (6), en particulier en forme de manchon, qui entoure la première tige intérieure (4) au moins partiellement et peut être relié ou serré avec celle-ci.

3. Electrode selon une des revendications 1 ou 2, **caractérisée en ce que** les deux tiges (4, 5) sont reliés entre elles de manière étanche aux liquides à l'extrémité proximale.

4. Electrode selon une des revendications 1 à 3, **caractérisée en ce que** le joint d'étanchéité pour la liaison étanche aux liquides des deux tiges est disposé au niveau du manchon d'accouplement (6), à l'intérieur de celui-ci.

5. Electrode selon une des revendications 1 à 4, **caractérisée en ce qu'**au moins une vis de serrage est prévue pour fixer la position relative des deux tiges (4, 5) en position d'utilisation.

6. Electrode selon une des revendications 1 à 5, **caractérisée en ce que** la première tige (4) présente un marquage (8) par rapport auquel la deuxième tige (5) peut être déplacée et réglée de manière contrôlée en direction longitudinale.

7. Electrode selon une des revendications 1 à 6, **caractérisée en ce que** la deuxième tige extérieure (5) peut tourner par rapport à la première tige (4).
